# EUROPEAN PATENT APPLICATION

(11) **EP 2 236 143 A1**
(43) Date of publication of application: **06.10.2010**
(21) Application number: 08868818.9
(22) Date of filing: 22.12.2008
(51) Int. Cl.: A61K 31/785, A61K 9/16, A61P 31/04

(54) **ANTIMICROBIAL AGENT FOR GRAM-POSITIVE BACTERIA**

(30) Priority: 27.12.2007 JP 2007337382
(71) Applicant: Public University Corporation Yokohama City University, Kanazawa-ku Yokohama-shi Kanagawa 236-0027 (JP)
(72) Inventor: SHIROTAKE, Shoichi, Yokohama-shi Kanagawa 236-0004 (JP)
(74) Representative: Wytenburg, Wilhelmus Johannes
(86) International application number: PCT/JP2008/073272
(87) International publication number: WO 2009/084494

(57) **Abstract**

A novel antibacterial agent for Gram-positive bacteria which disregards the drug-resistant mechanisms of bacteria is disclosed. The antibacterial agent contains as an effective ingredient particles having a particle diameter of not more than 5 µm, which particles are adhesive to cell wall of Gram-positive bacteria and not adhesive to mammalian cell membrane and substantially do not contain an active antibacterial ingredient effective against Gram-positive bacteria. By the antibacterial agent according to the present invention, the growth of the multidrug-resistant Gram-positive bacteria such as MRSA and VRE can be inhibited, as well as the occurrence of novel multidrug-resistant bacteria, which is a big problem in use of antibiotics, can be avoided.

## Description

### TECHNICAL FIELD

The present invention relates to an antibacterial agent for Gram-positive bacteria, comprising as an effective ingredient particles which substantially do not contain an active antibacterial ingredient effective against Gram-positive bacteria.

### BACKGROUND ART

Thanks to the development of many antibiotics in recent years, we today have the illusion that bacterial infectious diseases have been overcome. However, bacteria have acquired defense mechanisms against various antibiotics one by one, and thus survived to cause serious situations as a causative organism of nosocomial infection. Many of resistant bacteria such as methicillin-resistant *Staphylococcus aureus* (MRSA), vancomycin-resistant *Enterococcus* (VRE), vancomycin-resistant *Staphylococcus aureus* (VRSA) and multidrug-resistant *Pseudomonas aeruginosa* (MDRP) are resistant to almost all of the existing antibiotics, and therefore it is demanded worldwide to take a countermeasure quickly.

The resistant mechanism against antibiotics is specific to the respective antibiotics. Making free use of such various mechanisms, multidrug-resistant bacteria are surviving through multiple drug therapy. For the purpose of solving this problem, a variety of studies using various procedures such as exploration of a novel antibiotic, modification of the structure of antibiotics, improvement of drug delivery method, whole genome analysis of resistant bacteria, proteomics analysis, Multi-locus sequence typing, interferential action of indigenous bacteria and so on have started, the results of which are still insufficient. Measures to simultaneously block a plurality of the resistant mechanisms are required.

In order to improve the effect of pharmaceuticals by drug delivery system (DDS) or sustained release, studies of nano-encapsulation of drugs are now under way. For example, DDS by encapsulating a drug in cyanoacrylate polymer particles is known (Patent Literatures 1, 2 and Non-patent Literature 1). However, all the studies which have been carried out so far aim at DDS or sustained release of drugs. The antibacterial activity of nanoparticles *per se* is not known at all.
Patent Literature 1: JP 11-503148 A
Patent Literature 2: JP 2002-504526 A
Non-patent Literature 1: Christine Vauthier et al., Adv. Drug Deliv. Rev., 55, 519-548 (2003)

### DISCLOSURE OF THE INVENTION

### PROBLEMS TO BE SOLVED BY THE INVENTION

Accordingly, an object of the present invention is to provide novel means by which, overcoming a wide variety of antibiotic resistant mechanisms the resistant bacteria have acquired, an antibacterial activity can be exhibited against various multidrug resistant bacteria.

### MEANS FOR SOLVING THE PROBLEMS

The present inventors have studied a method for drug delivery by encapsulating antibacterial drugs in acrylate-based nanocapsules. Based on the know-how obtained from their studies, the inventors for the first time applied the particles alone which contained no antibiotics to bacteria and found the specific adhesion of the particles to the bacterial cell wall and the subsequent bacteriolysis, thereby completing the present invention.

That is, the present invention provides an antibacterial agent for Gram-positive bacteria, comprising as an effective ingredient particles having a particle diameter of not more than 5 µm, which particles are adhesive to cell wall of Gram-positive bacteria and not adhesive to mammalian cell membrane and substantially do not contain an active antibacterial ingredient effective against Gram-positive bacteria. The present invention also provides a method for inhibiting growth of a Gram-positive bacterium/bacteria, comprising bringing a Gram-positive bacterium/bacteria to be inhibited into contact with an effective amount of particles having a particle diameter of not more than 5 µm, which particles are adhesive to cell wall of Gram-positive bacteria and not adhesive to mammalian cell membrane and substantially do not contain an active antibacterial ingredient effective against Gram-positive bacteria. The present invention further provides use of particles having a particle diameter of not more than 5 µm, which particles are adhesive to cell wall of Gram-positive bacteria and not adhesive to mammalian cell membrane and substantially do not contain an active antibacterial ingredient effective against Gram-positive bacteria, for the production of an antibacterial agent for Gram-positive bacteria.

### EFFECTS OF THE INVENTION

By the present invention, a novel means for inhibiting bacterial growth by a mechanism completely different from the mechanism of known antibiotics was provided. Although there are antiseptics, which are an example of the germicide containing no antibiotics, they are for external application. Because of the toxicity, they cannot be administered to a living body, and thus cannot be used for treatment of infectious diseases. The particles used in the present invention can be prepared using a material which has been already used in practice with its safety for human health confirmed, and hence can be used for treatment of infectious diseases. The antibacterial agent according to the present invention is an agent which disregards the drug-resistance mechanisms bacteria have, and therefore can be applied to Gram-positive bacteria having a multidrug-resistance such as MRSA and VRE, as well as can avoid the occurrence of new multidrug resistant bacteria, which is a big problem in use of antibiotics. It is expected that the present invention will open up not only a way to treat infections by multidrug-resistant bacteria but also a completely novel field in research and development of antibacterial drugs, thereby providing an innovative development in the antibacterial drug studies.

### BRIEF DESCRIPTION OF THE DRAWINGS

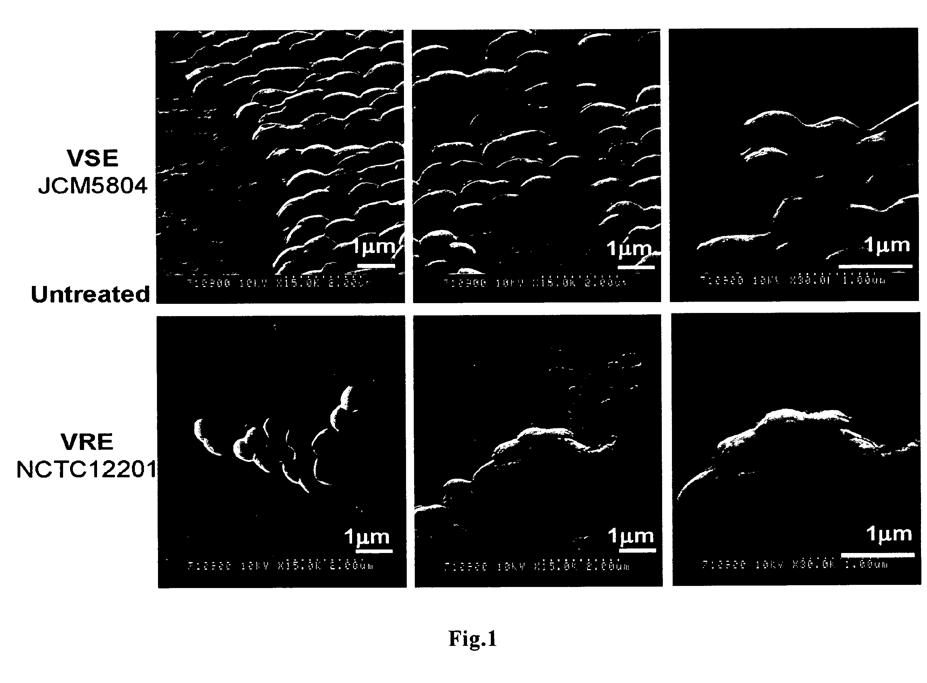

Fig. 1 shows SEM images of VSE and VRE.
Fig. 2 shows SEM images of VRE NCTC12201 treated with vancomycin.
Fig. 3 shows SEM images of VRE NCTC12201 treated with 0.01NHC1-Dex70+Glucose particles prepared in Production Example 1 (1-, 3- and 6-hour after the particle treatment).
Fig. 4 shows SEM images of VRE NCTC12201 and GTC02000 treated with 0.01NHCl-Dex70+Glucose particles prepared in Production Example 1 (1-hour after the particle treatment).
Fig. 5 shows SEM images of MRSA JCM8703 treated with 0.01NHCl-Dex70+Glucose particles prepared in Production Example 1 (1-, 3- and 6-hour after the particle treatment).
Fig. 6 is a graph showing a particle diameter distribution of each of particles prepared in Production Example 3.
Fig. 7 is a graph showing a zeta potential distribution of each of particles prepared in Production Example 3.

### BEST MODE FOR CARRYING OUT THE INVENTION

The antibacterial agent for Gram-positive bacteria according to the present invention contains as an effective ingredient particles which are adhesive to the cell wall of Gram-positive bacteria and not adhesive to the mammalian cell membrane, which particles substantially do not contain an active antibacterial ingredient effective against Gram-positive bacteria.

Particles used in the present invention have the characteristic feature that they are adhesive to the cell wall of Gram-positive bacteria but are not adhesive to the mammalian cell membrane. Whether particles are adhesive to the cell surface such as cell wall or cell membrane or not is judged as follows. Particles are suspended (6 µg/ml) in physiological saline or a culture medium such as Mueller Hinton Broth or the like, and using commercially available 24-well cell culture plate (usually having a well volume of 3.29 ml and a bottom growth area of 1.91 cm²), 1 ml of the suspension is added to about 10⁵ to 10⁶ cells/1 ml per well of Gram-positive bacterial cells or mammalian cells. After leaving the plate to stand for at least 1 hour, 1 ml per well of physiological saline is added with a micropipette, and the plate is slowly shaken twice or three times, followed by removing a supernatant washing fluid by aspiration. The same washing process is repeated another twice. The thus obtained cell sample is observed with an electron microscope. If particles are observed on the cell surface, it is judged that the particles are adhesive; if particles are not observed on the cell surface, it is judged that the particles are not adhesive. Although there are some cases where particles enter the inside of the cells by this kind of treatment, whether particles enter the inside of the cells or not is not considered in assessing the adhesiveness. As is well known, mammalian cells, not having a cell wall on their surface, are covered with a cell membrane, and the structure of this cell membrane consists of a lipid bilayer. Such a surface structure of mammalian cells is fundamentally the same among any animal species or any tissues. Therefore, the type of mammalian cells used in judging whether particles are adhesive to the mammalian cell membrane or not is not restricted. The mammalian cells used in the judgement may be tissue cell clumps collected from a living body or cells separated from the clumps, or may be an established cultured cell line. For example, as described in Examples below, adhesiveness to the mammalian cell membrane can be assessed by using cells such as a human cultured cell line HeLa cells, hamster CHO cells or the like. In the present specification, the adhesiveness of the particles that do not adhere to the mammalian cell membrane but adhere to the cell wall of Gram-positive bacteria is also referred to as "specific adhesion".

The particles substantially do not contain an active antibacterial ingredient effective against Gram-positive bacteria. The term "active antibacterial ingredient" means a chemical component which can biochemically affect metabolic pathway and/or physiological functions of Gram-positive bacteria and inhibit their growth, and specifically means a chemical component which can be used for inhibition of growth of Gram-positive bacteria such as antibiotics or the like. The term "substantially do not contain" means that particles do not contain any active antibacterial ingredients at all, or that even if they contain an active antibacterial ingredient(s), the amount of the contained active antibacterial ingredient(s) is so small that Gram-positive bacteria sensitive to the contained antibacterial ingredient(s) cannot be inhibited. In regard to the above-mentioned small amount by which the growth of sensitive bacteria cannot be inhibited, an amount of the active antibacterial ingredient(s) contained in a unit volume of the particles is defined as concentration in particles. When the growth of a sensitive Gram-positive bacterium cannot be inhibited by treating the bacterium with the active antibacterial ingredient(s) in form of not being encapsulated in particles at the same concentration as the above-defined concentration in particles, the concentration is considered to be an amount by which the growth of the sensitive Gram-positive bacterium cannot be inhibited. As particles used in the present invention, those not containing any active antibacterial ingredients such as antibiotics and the like at all are preferred.

The antibacterial agent according to the present invention is not an agent which utilizes the antibacterial activity of antibiotics, but an agent which causes bacteriolysis by adhesion of the particles to cell wall, thereby inhibiting bacterial growth. The detailed principle by which the above-described specific adhesion of the particles causes bacteriolysis is uncertain. Although the scope of the present invention is not bound by theory, the following is presented relating to the principle. That is, in the fundamental cell wall synthesis, UDP-MurNAc-pentapeptide is synthesized, and then bound to fatty acid and thereafter bound to GluNAc to form lipid-MurNAc(GluNAc)-pentapeptide. MurNAc therein is bound to GluNAc existing in a peptidoglycan which is being synthesized, and thus cell wall having a branching structure is built. As cell wall synthesis is executed on the external surface of the cell wall, it is thought that the particles adhere to the surface of bacterial cells to disturb the cell wall synthesis, thereby inducing bacteriolysis.

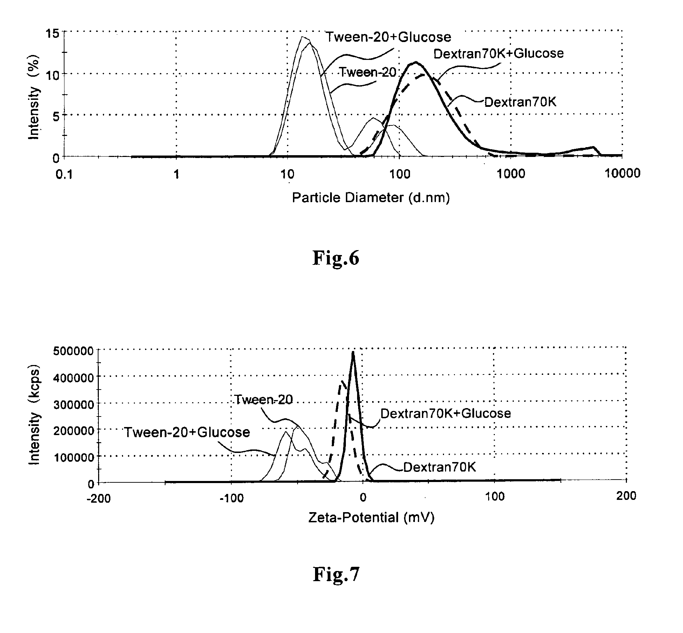

The antibacterial activity of the antibacterial agent according to the present invention is influenced by the particle diameter and the zeta-potential of the above-described particles (see, Examples below). The particle diameter of the particles used in the present invention is not more than 5 µm, preferably not more than 2 µm, more preferably not more than 1 µm. The lower limit of the particle diameter is not restricted, and in the case where an acrylate monomer is polymerized by, for example, such a method described in the following Examples to produce acrylate polymer particles, the diameter of the particles is usually about 7 nm or more (Fig. 6). The average particle diameter of all of the particles contained in the antibacterial agent is preferably 20 nm to 500 nm, especially preferably 20 nm to 300 nm. Zeta-potential is an electric potential of the surface of the particle, and serves as an index of the dispersibility of particles. It is preferred that the particles used in the present invention have a dispersibility good enough to prevent aggregation, because the particles act through adhesion to the surface of bacteria having a size of about several hundred nm to several µm. The zeta-potential of the particles used in the present invention is not restricted, and is preferably -3 mV to -80 mV. As shown in Examples below, the particle size and the zeta-potential may be easily measured with a commercially available apparatus using He·Ne laser (the model used in Examples: Zetasizer Nano, produced by Malvern Inst.UK). By using this apparatus, graphs of the particle diameter distribution and the zeta-potential distribution as shown in Figs. 6 and 7 are obtained, and the average particle diameter and the zeta-potential are calculated. The measurement principle by which this apparatus calculates an average particle diameter is a photon correlation method (dynamic light scattering) in which a particle diameter is calculated based on a fluctuation in the intensity of laser beam scattered by Brownian motion of the particles. The measurement principle by which this apparatus calculates a zeta-potential is an electrophoresis of the particles.

The above-described particles showing the specific adhesion are preferably particles whose repeating unit comprises a structure represented by Formula (I) below:

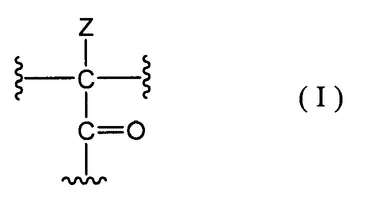

[wherein Z is an electron donating group]. Each of the wavy lines shown in the formula represents another moiety existing in the polymer structure, to which the structure represented by Formula (I) is bound. The structure represented by Formula (I) which has an electron donating group and a carboxyl group at the α-position has a high affinity to glycoproteins. Hence, this structure may have an affinity to the branching structure of the cell wall which consists of peptidoglycan and exists on the surface layer of bacterial cells, and may preferably exhibit the specific adhesion property described above.

The electron donating group Z is preferably a cyano group, an amino group or an imino group, and more preferably a cyano group.

Examples of the structure represented by Formula (I) include, but not limited to, a structure represented by Formula (II) below.

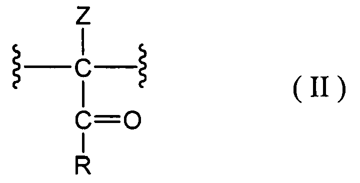

[wherein Z represents the same meaning as described in Formula (I) above; R is hydrogen, a hydroxy group, C₁-C₁₀ alkyl group, C₁-C₁₀ alkoxy group, C₆-C₁₅ aryl group, C₆-C₁₅ aryloxy group, C₇-C₁₆ aralkyl group or a C₇-C₁₆ aralkyloxy group; one or more carbon atoms constituting a carbon chain in R are optionally replaced by nitrogen, sulfur and/or oxygen]

Among the structures represented by Formula (II), those wherein R is a hydroxy group or a C₁-C₁₀ alkoxy group are preferred, and those wherein R is a n-butoxy group are especially preferred. Preferred examples of Z are described above. Therefore, among the structures represented by Formula (II), a structure wherein Z is a cyano group and R is a n-butoxy group is especially preferred.

Among the particles used in the present invention, polymer particles whose repeating unit comprises a structure represented by Formula (II) above may be produced by polymerizing a monomer represented by the following Formula (III):

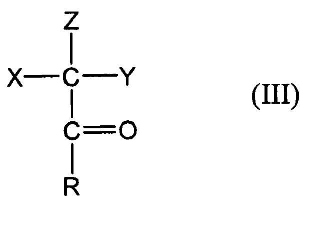

[wherein Z and R represent the same meanings as in Formula (II) above; X and Y each independently represent an atomic group having a carbon-carbon double bond in its terminal, or X and Y together form a methylidene group] in the presence of a saccharide(s) having a hydroxy group(s) and/or polysorbate(s). Examples of the monomer having such a structure include acrylate monomers. Various acrylate monomers are commercially available, and such commercial products may be preferably used. Other monomers may be easily prepared from, for example, a commercially-available acrylate monomer by using a conventional method well-known in the chemical synthesis field.

Among the monomers represented by Formula (III), monomers wherein R is a hydroxy group or a C₁-C₁₀ alkoxy group, i.e., acrylate monomers substituted by an electron donating group Z, are preferred. The electron donating group Z is, as described above, preferably a cyano group, an amino group or an imino group, and more preferably a cyano group. Thus, as the above-described monomer, cyanoacrylate monomers are especially preferred. Preferred examples of the cyanoacrylate monomer include n-butyl-2-cyanoacrylate (nBCA) represented by the following formula. As nBCA has been conventionally used as an adhesive for wound closure in the field of surgery, its safety for human health has been confirmed.

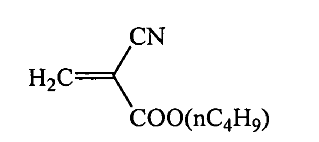

The above-described monomer can be polymerized by anionic polymerization. Although not restricted, in the anionic polymerization, a saccharide(s) and/or polysorbate(s) may be used for initiation and stabilization of the polymerization. Therefore, "polymer particles" which may be used in the present invention include those containing a polymerization initiator/stabilizer such as a saccharide(s) and/or polysorbate(s). By using a saccharide(s) and/or polysorbate(s), uniform particles with little irregularity in particle diameter can be preferably prepared.

Saccharides are not restricted, and may be any of monosaccharides having a hydroxyl group(s), disaccharides having a hydroxyl group(s) and polysaccharides having a hydroxyl group(s). Examples of the monosaccharide include glucose, mannose, ribose, fructose and the like. Examples of the disaccharide include maltose, trehalose, lactose, sucrose and the like. Examples of the polysaccharide include dextran, which is used for polymerization of conventional cyanoacrylate polymer particles, mannan and the like. These saccharides may be either a cyclic form or a chain form, and in case of cyclic form, saccharides may be either a pyranose form or a furanose form. Saccharides have various isomers, and any of such isomers may be used in the present invention. Usually, monosaccharides exist in the form of pyranose or furanose, and such monosaccharides are α- or β-lined to form a disaccharide. Saccharides in such an ordinary form may be used without modification. Polysorbates are not restricted, and may be any of the known Tween series surfactants such as polyoxyethylene sorbitan monolaurate (trade name: Tween 20), polyoxyethylene sorbitan monooleate (trade name: Tween 80) and the like. Monosaccharides, disaccharides and polysaccharides and polysorbates may be used individually, or two or more of these may be used in combination. Among the saccharides and polysorbates described above, glucose, ribose, lactose, trehalose, dextran and Tween 20 (trade name) are preferred. As for dextran, one having an average molecular weight of not less than 70,000 is preferred. The upper limit of the molecular weight of dextran is not restricted, and the molecular weight is usually not more than about 500,000.

As the solvent for the polymerization, water is usually used. Because the anionic polymerization is initiated by hydroxide ion, the rate of polymerization is influenced by pH of the reaction solution. When pH of the reaction solution is high, polymerization proceeds rapidly because of a high concentration of hydroxide ion. When pH is low, polymerization proceeds slowly. An appropriate polymerization rate is usually attained under an acidic condition of about pH 2 to pH 4. The acid added to the reaction solution in order to acidify it is not restricted, and hydrochloric acid may be preferably used as it does not have a bad influence on the reaction and vaporizes after the reaction.

The concentration of the monomer in the polymerization reaction solution at the start of the reaction is not restricted, and is usually about 0.5 v/v% to 2.0 v/v%, preferably about 0.8 v/v% to 1.5 v/v%. In the case where a saccharide(s) and/or polysorbate(s) is(are) used for the polymerization reaction, the concentration of the saccharide(s) and/or polysorbate(s) (in the case where a plurality of kinds thereof are used, the total concentration thereof) is not restricted, and is usually about 0.5% to 10%, preferably about 0.75% to 7.5%. As used herein, the concentration of saccharides means w/v%, and the concentration of polysorbates means v/v%. For example, in the case where a single saccharide is used, the concentration ranges described above mean "0.5 w/v% to 10 w/v%" and "0.75 w/v% to 7.5 w/v%", respectively. In the case where 5 w/v% of saccharide and 1 v/v% of polysorbate are used in combination, the total concentration thereof is expressed as 6%. It should be noted that, in the case where a monosaccharide(s) (e.g. glucose) is(are) used alone, the monosaccharide(s) is(are) preferably used at a concentration of about 2.5 w/v% to 10 w/v%. The reaction temperature is not restricted, and the reaction is preferably carried out at room temperature because it is simple. The reaction time is not restricted, and is usually about 15 minutes to 4 hours, preferably about 30 minutes to 3 hours. The polymerization reaction is preferably carried out under stirring. As the particles are usually used as neutral particles, it is preferred to neutralize the reaction solution by adding thereto a base such as aqueous sodium hydroxide solution or the like after completion of the reaction.

By the polymerization reaction described above, a monomer is anionically polymerized to form polymer particles. The formed particles can be collected by a conventional method such as centrifugal ultrafiltration or the like. By the above-described method, polymer particles having an average particle diameter of about 20 nm to 500 nm, preferably about 20 nm to 300 nm, and a zeta-potential of about -3 mV to -80 mV can be obtained, and these particles may be preferably used as the above-described particles in the present invention. The size of the particles can be regulated by regulating the monomer concentration in the reaction solution and/or the reaction time. In the case where a saccharide(s) and/or a polysorbate(s) is(are) used as a polymerization initiator/stabilizer, the particle size can also be regulated by changing the concentration and/or the kind of the polymerization initiator/stabilizer (see, Examples below).

Bacteria against which the antibacterial agent of the present invention exhibits its antibacterial activity is Gram-positive bacteria. The type of bacteria is not restricted as long as they are Gram-positive, and the antibacterial agent of the present invention can inhibit the growth of not only bacteria sensitive to antibiotics such as MSSA (methicillin-sensitive *Staphylococcus aureus*) and VSE (vancomycin-sensitive *Enterococcus*) but also multidrug-resistant bacteria such as MRSA (methicillin-resistant *Staphylococcus aureus*) and VRE (vancomycin-resistant *Enterococcus*). Independently of the resistance of Gram-positive bacteria to antibiotics, the antibacterial agent of the present invention lyses Gram-positive bacteria by the specific adhesion of the particles described above, thereby inhibiting their growth. Thus, the antibacterial agent of the present invention has no risk of occurrence of new multidrug-resistant bacteria as well as can inhibit the growth of the existing multidrug-resistant bacteria, which is very advantageous in the clinical use.

The antibacterial agent of the present invention may consist of the above-described particles alone or, in the case where the agent is used for treatment of Gram-positive bacterial infections, may be formulated into a form suitable for the administration method by mixing the particles with known excipients, carriers and/or the like. The particles may consist of a single kind of particles alone, or two or more kinds of particles may be used in combination. Examples of the method for administration of the antibacterial agent according to the present invention include parenteral administration such as subcutaneous, intramuscular, intraperitoneal, intraarterial, intravenous and intrarectal administration and oral administration. Specifically, for example, the particles may be suspended in physiological buffered saline and parenterally administered by injection or the like, or the particles may be orally administered as a capsule or syrup, but the administration method is not restricted thereto. In the case where the antibacterial agent of the present invention is used for sterilization of medical instruments, the particles may be, for example, dispersed in water, an alcohol solvent or the like to immerse medical instruments therein. By administering the above-described particles to a body or bringing the above-described particles into contact with instruments or the like, the particles are brought into contact with Gram-positive bacteria to be inhibited, thereby attaining inhibition of the growth of Gram-positive bacteria.

The level (MIC value and MBC value) of antibacterial activity of the above-described particles is influenced by particle diameter and zeta-potential of the particles and also varies depending on the type of resistant bacteria. Usually, the particles exhibit *in vitro* antibacterial activity at a concentration of about 0.01 mg/ml to 25 mg/ml. For example, the particles consisting of cyanoacrylate polymer described in Examples below exhibit *in vitro* antibacterial activity at a concentration of about 0.025 mg/ml to 6.4 mg/ml against various *Staphylococcus aureus* strains and *Enterococcus* strains used in the following Examples. In the case where the antibacterial agent of the present invention is used for treatment of infections, the above-described particles may be usually administered, but not limited to, at a single dose of about 0.1 g to 100 g, particularly about 0.1 g to 25 g to an adult.

### EXAMPLES

The present invention will now be described more concretely by way of an example thereof. However, the present invention is not restricted to the example below.

### Production Example 1

### Production of Cyanoacrylate Polymer Particles

### (Dextran, and Dextran+Glucose Reaction Systems)

Cyanoacrylate polymer particles were produced using nBCA (Histoacryl (registered trademark), Braun, Melsungen, Germany) as a monomer. As a polymerization initiator/stabilizer, dextran alone or dextran + glucose were used. The average molecular weight of dextran used herein was 70K (Dex70).

In 100 ml of hydrochloric acid, (1) 1 g of Dex70 or (2) 1 g of Dex70 and 5 g of glucose were dissolved. The concentration of the used hydrochloric acid was 0.05 N, 0.01 N, and 0.001 N. To the respective solutions, 1.2 mL of nBCA (concentration in the reaction solution: 1.2 v/v%) was added under stirring, and the resulting solutions were stirred (600 rpm) at room temperature for 2 hours to carry out polymerization reaction. Thereafter, 0.1 N NaOH was added to the reaction solutions to neutralize them, and the resulting solutions were stirred for 15 minutes. The reaction solutions were filtered through a Milex filter (trade name, MILLIPORE) with a size of 5 µm, and the filtrates were filtered by centrifugal filtration using a Centriprep filter (trade name, MILLIPORE) at 3000 rpm/15 minutes. DW was added to the liquids which did not pass through a Centriprep filter to suspend the liquids therein, and the resulting suspensions were centrifuged at 3000 rpm/15 minutes. The same operation was repeated 3 times to obtain polycyanoacrylate particles (0.05NHCl-Dex70, 0.05NHCl-Dex70+Glucose, 0.01NHC1-Dex70, 0.01NHCl+Glucose, 0.001NHCl-Dex70, 0.001NHCl-Dex70+Glucose).

The average particle diameter and the zeta-potential of the obtained particles were measured with a commercially available measurement apparatus using He·Ne laser scattering (model: Zetasizer Nano, produced by Malvern Inst.UK). The results are shown in Table 1.

**Table 1**

| | Average particle diameter (nm) | Peak1 (nm) Peak2 (nm) | Peak width (nm) | Zeta- potential (mV) |
|---|---|---|---|---|
| 0.05NHCl-Dex70 | 187 | 233(94.0%) | 179 | -28.3 |
| | PdI:0.346 | 4640( 6.0%) | 820 | |
| 0.05NHCl-Dex70+Glucose | 179 | 195(98.5%) | 69.0 | -58.1 |
| | PdI:0.151 | 4910(1.4%) | 659 | |
| 0.01NHCl-Dex70 | 134 | 140(89.5%) | 58.5 | -14.3 |
| | PdI:0.250 | 1950(10.5%) | 977 | |
| 0.01NHCl-Dex70+Glucose | 150 | 289(100%) | 321 | -13.0 |
| | PdI:0.331 | | | |
| 0.001NHCI-Dex70 | 178 | 187(100%) | 44.7 | -22.5 |
| | PdI:0.012 | | | |
| 0.001NHCl-Dex70+Glucose | 155 | 165(100%) | 43.2 | -19.4 |
| | PdI:0.054 | | | |

### Example 1

### Antibacterial Activity of Cyanoacrylate Polymer Particles

### (Dextran, and Dextran+Glucose Reaction Systems)

The antibacterial activity (minimum inhibitory concentration (MIC) and minimum bactericidal concentration (MBC)) of the particles prepared in Production Example 1 was examined against various bacterial strains. Ampicillin (ABPC) was used as a positive control. The measurement was carried out in accordance with a broth microdilution method (NCCLS). That is, 256 µg/ml of ABPC solution and 6.4 mg/ml of particle suspension were prepared as undiluted solutions (1-fold dilution), and each undiluted solution was 2-fold serially diluted up to 2048-fold to prepare 12 concentrations in total, which were used for assessing the antibacterial activity. These serial dilutions were placed in a 96-well plate, and a bacterial strain was added to each well at an amount of 10⁵ CFU/ml, followed by incubation at 35°C for 18 hours. In the case where turbidity was visually observed, it was judged that the bacterial strain grew. The lowest concentration at which the bacterial growth was not observed was defined as MIC. A solution was collected from the well in which bacterial growth was not observed, added to an antibiotic-free medium, and then incubated at 35°C for 18 hours, followed by visually observing existence of turbidity. The lowest concentration at which turbidity was not observed was defined as MBC. The results are shown in Tables 2 to 4. Values in the tables are: ABPC, µg/ml; polymer particles, mg/ml. Numbers in the parentheses indicate the dilution factor.

**Table 2**

| MIC and MBC Values against Standard Strains of MSSA and MRSA | | | | | | | | |
|---|---|---|---|---|---|---|---|---|
| | MSSA | | MSSA | | MRSA | | MRSA | |
| | JCM2874 | | ATCC6538 | | N315 | | JCM8703 | |
| | MIC | MBC | MIC | MBC | MIC | MBC | MIC | MBC |
| ABPC alone | 2 | 2 | 0.063 | 0.063 | 32 | 32 | 64 | 64 |
| 0.05NHCl | 0.1 | 3.2 | 0.2 | 0.1 | 0.2 | 3.2 | 0.1 | 0.2 |
| Dex70 | (x64) | (x2) | (x128) | (x64) | (x32) | (x2) | (x64) | (x32) |
| 0.05NHCl | 0.1 | 3.2 | 0.1 | 0.2 | 0.2 | 3.2 | 0.1 | 0.2 |
| Dex+Glu | (x64) | (x2) | (x64) | (x32) | (x32) | (x2) | (x64) | (x32) |
| 0.01NHCl | 0.1 | 0.1 | 0.1 | 0.1 | 0.1 | 0.2 | 0.1 | 0.1 |
| Dex70 | (x64) | (x64) | (x64) | (x64) | (x64) | (x32) | (x64) | (x64) |
| 0.01NHCl | 0.1 | 0.1 | 0.1 | 0.1 | 0.1 | 0.1 | 0.1 | 0.1 |
| Dex70+Glu | (x64) | (x64) | (x64) | (x64) | (x64) | (x64) | (x64) | (x64) |
| 0.001NHCl | 0.025 | 0.8 | 0.05 | 1.6 | 0.1 | 3.2 | 0.1 | 0.2 |
| Dex70 | (x256) | (x8) | (x128) | (x4) | (x64) | (x2) | (x64) | (x32) |
| 0.001NHCl | 0.025 | 0.8 | 0.025 | 1.6 | 0.2 | 3.2 | 0.1 | 0.1 |
| Dex70+Glu | (x256) | (x8) | (x256) | (x4) | (x128) | (x2) | (x64) | (x64) |

**Table 3**

| MIC and MBC Values against MRSA Clinical Isolates | | | | | | | | |
|---|---|---|---|---|---|---|---|---|
| | 1801 blaZ(-) | | 2022 blaZ(-) | | 2005 blaZ(+) | | 2526 blaZ(+) | |
| | MIC | MBC | MIC | MBC | MIC | MBC | MIC | MBC |
| ABPC alone | 16 | 16 | 16 | 16 | 64 | 64 | 64 | 64 |
| 0.05NHCl | 0.1 | 0.1 | 0.05 | 0.025 | 0.1 | 0.2 | 0.1 | 3.2 |
| Dex70 | (x64) | (x64) | (x128) | (x256) | (x64) | (x32) | (x64) | (x2) |
| 0.05NHCl | 0.1 | 0.1 | 0.1 | 0.05 | 0.2 | 0.2 | 0.05 | 3.2 |
| Dex+Glu | (x64) | (x64) | (x64) | (x128) | (x32) | (x32) | (x128) | (x2) |
| 0.01NHCl | 0.2 | 0.2 | 0.2 | 0.2 | 0.1 | 0.1 | 0.4 | 3.2 |
| Dex70 | (x32) | (x32) | (x32) | (x32) | (x64) | (x64) | (x 16) | (x2) |
| 0.01NHCl | 0.1 | 0.2 | 0.1 | 0.2 | 0.1 | 0.1 | 0.4 | 1.6 |
| Dex70+Glu | (x64) | (x32) | (x64) | (x32) | (x64) | (x64) | (x 16) | (x4) |
| 0.001NHCl | 0.1 | 0.1 | 0.1 | 0.4 | 0.05 | 0.2 | 0.05 | 0.05 |
| Dex70 | (x64) | (x64) | (x64) | (x 16) | (x128) | (x32) | (x128) | (x128) |
| 0.001NHCl | 0.1 | 0.1 | 0.1 | 0.4 | 0.05 | 0.1 | 0.05 | 0.05 |
| Dex70+Glu | (x64) | (x64) | (x64) | (x16) | (x128) | (x64) | (x128) | (x128) |

**Table 4**

| MIC and MBC against VSE and VRE | | | | | | |
|---|---|---|---|---|---|---|
| | VSE5804 | | VRE | | VRE | |
| | | | GTC02000 | | NCTC12201 | |
| | MIC | MBC | MIC | MBC | MIC | MBC |
| ABPC alone | 0.5 | 0.5 | 1 | 1 | 1 | 1 |
| 0.05NHCl | 0.2 | 0.2 | 0.2 | 1.6 | 0.2 | 0.2 |
| Dex70 | (x32) | (x32) | (x32) | (x4) | (x32) | (x32) |
| 0.05NHCl | 0.2 | 0.2 | 0.2 | 1.6 | 0.2 | 0.2 |
| Dex+Glu | (x32) | (x32) | (x32) | (x4) | (x32) | (x32) |
| 0.01NHCl | Not determined | Not determined | 0.1 | 1.6 | 0.2 | 0.2 |
| Dex70 | | | (x64) | (x4) | (x32) | (x32) |
| 0.01NHCl | Not determined | Not determined | 0.1 | 0.8 | 0.1 | 0.1 |
| Dex70+Glu | | | (x64) | (x8) | (x64) | (x64) |
| 0.001NHCl | 0.1 | 0.1 | 0.1 | 1.6 | 0.1 | 0.1 |
| Dex70 | (x64) | (x64) | (x64) | (x4) | (x64) | (x64) |
| 0.001NHCl | 0.1 | 0.1 | 0.1 | 1.6 | 0.1 | 0.1 |
| Dex70+Glu | (x64) | (x64) | (x64) | (x4) | (x64) | (x64) |

When the dextran reaction system and the dextran + glucose reaction system were compared, the antibacterial activities of the obtained particles were at about the same level. In regard to the concentration of hydrochloric acid, 0.01 N tended to provide particles with a higher antibacterial activity than 0.05 N.

The MIC against *Staphylococcus aureus* was 0.025 to 0.4 mg/ml, and the antibacterial activities against MSSA and MRSA were at about the same level. The MBC was different among the strains.

The MIC against *Enterococcus* was 0.1 to 0.2 mg/ml, and no difference was observed between the antibacterial activities against VSE and VRE. In some cases, a significant difference was observed in MBC between the strains (1.6 mg/ml and 0.1 mg/ml).

### Production Example 2

### Production of Cyanoacrylate Polymer Particles

### (Glucose, Ribose, Lactose, and Trehalose Reaction Systems)

Cyanoacrylate polymer particles were produced using nBCA (Histoacryl (registered trademark), Braun, Melsungen, Germany) as a monomer. As a polymerization initiator/stabilizer, glucose, ribose, lactose or trehalose was used.

The same procedure as in Production Example 1 was carried out except that 5 g of any one of the above-described monosaccharides and disaccharides was used as a saccharide to produce polycyanoacrylate particles (0.05NHCl-Glucose, 0.05NHCl-Ribose, 0.05NHCl-Lactose, 0.05NHCl-Trehalose, 0.01NHCl-Glucose, 0.01NHCl-Ribose, 0.01NHCl-Lactose, 0.01NHCl-Trehalose, 0.001NHCl-Glucose).

He·Ne laser light scattering analysis was performed using a commercially available measurement apparatus (supra) to measure the average particle diameter and the zeta-potential of the obtained particles. The results are shown in Table 5.

**Table 5**

| | Average particle diameter (nm) | Peak1 (nm) | Peak width (nm) | Zeta- potential (mV) |
|---|---|---|---|---|
| 0.05NHCl-Glucose | 286 PdI:0.152 | 301(100%) . | 95.2 | -75.3 |
| 0.05NHCl-Ribose | 256 PdI:0.134 | 273(100%) | 81.7 | -75.4 |
| 0.05NHCl-Lactose | 254 PdI:0.088 | 281(100%) | 91.0 | -70.2 |
| 0.05NHCl-Trehalose | 273 PdI:0.119 | 313(100%) | 117 | -65.5 |
| 0.01NHCl-Glucose | 205 PdI:0.046 | 217(100%) | 53.3 | -57.5 |
| 0.01NHCl-Ribose | 266 PdI:0.150 | 319(100%) | 145 | -63.6 |
| 0.01NHCl-Trehalose | 183 PdI:0.061 | 194(100%) | 47.3 | -57.6 |
| 0.01NHCl-Lactose | 225 PdI:0.028 | 235(100%) | 51.3 | -55.8 |
| 0.001NHCl-Glucose | 150 PdI:0.057 | 160(100%) | 42.5 | -50.6 |

### Example 2

### Antibacterial Activity of Cyanoacrylate Polymer Particles

### (Glucose, Ribose, Lactose, and Trehalose Reaction Systems)

In the same manner as in Example 1, the antibacterial activity (minimum inhibitory concentration (MIC) and minimum bactericidal concentration (MBC)) of the particles prepared in Production Example 2 was examined against various bacterial strains. The results are shown in Tables 6 to 8.

**Table 6**

| MIC and MBC Values against Standard Strains of MSSA and MRSA | | | | | | | | |
|---|---|---|---|---|---|---|---|---|
| | MSSA | | MSSA | | MRSA | | MRSA | |
| | JCM2874 | | ATCC6538 | | N315 | | JCM8703 | |
| | MIC | MBC | MIC | MBC | MIC | MBC | MIC | MBC |
| ABPC | 2 | 2 | ≤0.125 | ≤0.125 | 32 | 32 | 64 | 64 |
| alone | | | | | | | | |
| 0.05NHCl- | 3.2 | 3.2 | 3.2 | 3.2 | ≥6.4 | ≥6.4 | 3.2 | ≥6.4 |
| Glucose | (x2) | (x2) | (x2) | (x2) | | | (x2) | |
| 0.05NHCl- | 3.2 | 3.2 | 3.2 | 3.2 | 3.2 | 3.2 | 3.2 | 3.2 |
| Ribose | (x2) | (x2) | (x2) | (x2) | (x2) | (x2) | (x2) | (x2) |
| 0.05NHCl- | 3.2 | 3.2 | 3.2 | ≥6.4 | 3.2 | ≥6.4 | 3.2 | ≥6.4 |
| Lactose | (x2) | (x2) | (x2) | | (x2) | | (x2) | |
| 0.05NHCl- | 3.2 | ≥6.4 | 3.2 | ≥6.4 | 3.2 | ≥6.4 | 3.2 | ≥6.4 |
| Trehalose | (x2) | | (x2) | | (x2) | | (x2) | |
| 0.01NHCl- | 0.8 | 0.8 | 1.6 | 3.2 | 0.8 | 0.8 | 1.6 | 1.6 |
| Glucose | (x8) | (x8) | (x4) | (x2) | (x8) | (x8) | (x4) | (x4) |
| 0.01NHCl- | 0.4 | 0.8 | 0.8 | 1.6 | 1.6 | 1.6 | 1.6 | 1.6 |
| Ribose | (x16) | (x8) | (x8) | (x4) | (x4) | (x4) | (x4) | (x4) |
| 0.01NHCl- | 6.4 | 6.4 | 3.2 | 6.4 | 6.4 | 6.4 | 6.4 | 6.4 |
| Lactose | (x1) | (x1) | (x2) | (x1) | (x1) | (x1) | (x1) | (x1) |
| 0.01NHCl- | 3.2 | 3.2 | 0.8 | 1.6 | 1.6 | 3.2 | 6.4 | 6.4 |
| Trehalose | (x2) | (x2) | (x8) | (x4) | (x4) | (x2) | (x1) | (x1) |
| 0.001NHCl- | 0.2 | 0.2 | 0.4 | 0.4 | 0.4 | 1.6 | 0.4 | 0.8 |
| Glucose | (x32) | (x32) | (x16) | (x16) | (x16) | (x4) | (x16) | (x8) |

**Table 7**

| MIC and MBC Values against MRSA Clinical Isolates | | | | | | | | |
|---|---|---|---|---|---|---|---|---|
| | 1801 | | 2022 | | 2005 | | 2526 | |
| | blaZ(-) | | blaZ(-) | | blaZ(+) | | blaZ(+) | |
| | MIC | MBC | MIC | MBC | MIC | MBC | MIC | MBC |
| ABPC | 16 | 16 | 16 | 16 | 64 | 64 | 64 | 64 |
| alone | | | | | | | | |
| 0.05NHCl- | 3.2 | 3.2 | 3.2 | 3.2 | 3.2 | 3.2 | 3.2 | ≥6.4 |
| Glucose | (x2) | (x2) | (x2) | (x2) | (x2) | (x2) | (x2) | |
| 0.05NHCl- | 3.2 | 3.2 | 3.2 | 3.2 | 3.2 | 3.2 | 3.2 | 3.2 |
| Ribose | (x2) | (x2) | (x2) | (x2) | (x2) | (x2) | (x2) | (x2) |
| 0.05NHCl- | 3.2 | 3.2 | 3.2 | 3.2 | 3.2 | 3.2 | 3.2 | ≥6.4 |
| Lactose | (x2) | (x2) | (x2) | (x2) | (x2) | (x2) | (x2) | |
| 0.05NHCl- | 3.2 | 3.2 | 3.2 | 3.2 | 3.2 | 3.2 | 3.2 | ≥6.4 |
| Trehalose | (x2) | (x2) | (x2) | (x2 | (x2) | (x2) | (x2) | |
| 0.01NHCl- | 0.8 | 0.8 | 0.8 | 0.8 | 0.8 | 0.8 | 1.6 | 3.2 |
| Glucose | (x8) | (x8) | (x8) | (x8) | (x8) | (x8) | (x4) | (x2) |
| 0.01NHCl- | 0.8 | 0.4 | 0.4 | 0.2 | 0.4 | 0.8 | 0.8 | 1.6 |
| Ribose | (x8) | (x16) | (x16) | (x32) | (x 16) | (x8) | (x8) | (x4) |
| 0.01NHCl- | 1.6 | 3.2 | 1.6 | 3.2 | 6.4 | 6.4 | 6.4 | 6.4 |
| Lactose | (x4) | (x2) | (x4) | (x2) | (x1) | (x1) | (x1) | (x1) |
| 0.01NHCl- | 3.2 | 3.2 | 3.2 | 3.2 | 1.6 | 3.2 | 3.2 | 3.2 |
| Trehalose | (x2) | (x2) | (x2) | (x2) | (x4) | (x2) | (x2) | (x2) |
| | 0.4 | 0.4 | 0.4 | 0.4 | 0.4 | 0.4 | 0.8 | 0.4 |
| 0.001NHOl- Glucose | (x16) | (x16) | (x16) | (x16) | (x16) | (x16) | (x8) | (x16) |

**Table 8**

| MIC and MBC against VRE | | | | |
|---|---|---|---|---|
| | VRE | | VRE | |
| | GTC02000 | | NCTC12201 | |
| | MIC | MBC | MIC | MBC |
| ABPC | 1 | 1 | 1 | 1 |
| alone | | | | |
| 0.05NHCl- | >6.4 | >6.4 | >6.4 | >6.4 |
| Glucose | (x1) | (x1) | (x1) | (x1) |
| 0.05NHCl- | 3.2 | >6.4 | 3.2 | 3.2 |
| Ribose | (x2) | (x1) | (x2) | (x2) |
| 0.05NHCl- | >6.4 | >6.4 | >6.4 | >6.4 |
| Lactose | (x1) | (x1) | (x1) | (x1) |
| 0.05NHCl- | >6.4 | >6.4 | >6.4 | >6.4 |
| Trehalose | (x1) | (x1) | (x1) | (x1) |
| 0.01NHCl- | 1.6 | >6.4 | 1.6 | 1.6 |
| Glucose | (x4) | (x1) | (x4) | (x4) |
| 0.01NHCl- | 3.2 | >6.4 | 3.2 | 3.2 |
| Ribose | (x2) | (x1 | (x2) | (x2) |
| 0.01NHCl- | 1.6 | >6.4 | 1.6 | >6.4 |
| Lactose | (x4) | (x1) | (x4) | (x1) |
| 0.01NHCl- | 3.2 | 3.2 | 3.2 | >6.4 |
| Trehalose | (x2) | (x2) | (x2) | (x1) |

By comparison between the monosaccharide system and the disaccharide system, it was revealed that polymer particles obtained in the monosaccharide system had a higher antibacterial activity. Focusing on the influence of hydrochloric acid concentration, the level of antibacterial activity of the obtained polymer particles was 0.01N HCl system > 0.05N HCl system in the monosaccharide system, and 0.01N HCl system≤ 0.05N HCl system in the disaccharide system.

The MIC against *Staphylococcus aureus* was 0.4 to 3.2 mg/ml, and the antibacterial activities against MSSA and MRSA were at about the same level. The MBC was different among the strains.

The MIC against *Enterococcus* was 1.6 to 3.2 mg/ml, and no difference was observed between the antibacterial activities against VSE and VRE (data not shown). In some cases, a significant difference was observed in MBC between the strains (1.6 mg/ml and >6.4 mg/ml).

### Production Example 3

### Production of Cyanoacrylate Polymer Particles

### (Tween 20, and Tween 20+Glucose Reaction Systems)

Cyanoacrylate polymer particles were produced using nBCA (Histoacryl (registered trademark), Braun, Melsungen, Germany) as a monomer. As a polymerization initiator/stabilizer, Tween 20 or Tween 20 + glucose were used. The polymerization reaction was carried out for 1 hour, and Dex70 and Dex70 + glucose, which were examined in Production Example 1, were examined herein again.

In 100 ml of 0.01 N hydrochloric acid, (1) 1 ml of Tween 20, (2) 1 ml of Tween 20 and 5 g of glucose, (3) 1 g of Dex70 or (4) 1 g of Dex70 and 5 g of glucose were dissolved. To the respective solutions, 1.2 mL of nBCA (concentration in the reaction solution: 1.2 v/v%) was added under stirring, and the resulting solutions were stirred (600 rpm) at room temperature for 1 hour to carry out polymerization reaction. Thereafter, 0.1 N NaOH was added to the reaction solutions to neutralize them, and the resulting solutions were stirred for 15 minutes. The reaction solutions were filtered through a Milex filter (trade name, MILLIPORE) with a size of 5 µm, and the filtrates were filtered by centrifugal filtration using a Centriprep filter (trade name, MILLIPORE) at 3000 rpm/15 minutes. DW was added to the liquids which did not pass through a Centriprep filter to suspend the liquids therein, and the resulting suspensions were centrifuged at 3000 rpm/15 minutes. The same operation was repeated 4 times to obtain polycyanoacrylate particles (0.01NHCl-Tw20, 0.01NHCl-Tw20+Glucose, 0.01NHCl-Dex70(lhr),0.01NHCl-Dex70+Glu(lhr)).

He·Ne laser light scattering analysis was performed using a commercially available measurement apparatus (supra) to measure the average particle diameter and the zeta-potential of the obtained particles. The results are shown in Table 9. Graphs of a particle diameter distribution and a zeta-potential distribution obtained by the analysis using the apparatus are shown in Fig. 6 and Fig. 7, respectively.

**Table 9**

| | Average particle diameter (nm) | Peak1 (nm) Peak2 (nm) | Peak width (nm) | Zeta- potential (mv) |
|---|---|---|---|---|
| 0.01NHCl-Tw20 | 33.7 | 17.1 (81.3%) | 5.58 | -43.9 |
| | PdI:0.119 | 89.9(18.7%) | 23.7 | |
| 0.01NHCl-Tw20+Glucose | 88.6 | 15.5 (79.1%) | 4.85 | -53.0 |
| | PdI:0.151 | 59.0 (20.9%) | 14.3 | |
| 0.01NHCl-Dex70(lhr) | 163 | 211(95.2%) | 194 | -6.31 |
| | PdI:0.250 | 4140(4.2%) | | |
| 0.01NHCl-Dex70+Glu(1hr) | 147 | | 104 | -14.9 |
| | PdI:0.208 | 192(100%) | | |

### Example 3

Antibacterial Activity of Cyanoacrylate Polymer Particles
(Tween 20, and Tween 20 + Glucose Reaction Systems)
In the same manner as in Example 1, the antibacterial activity (minimum inhibitory concentration (MIC)) of the particles produced in Production Example 3 was examined against various bacterial strains. The results are shown in Table 10.

**Table 10**

| MIC Values against Each Strain | | | | |
|---|---|---|---|---|
| | ATCC6538 | N315 | GTC0200 | NCTC12201 |
| | MSSA | MRSA | VRE | VRE |
| ABPC alone | 0.063 | 8 | 1 | 2 |
| 0.01NHCl- | 0.09 | 0.05 | 0.09 | 0.09 |
| Tw20 | (x64) | (x128) | (x64) | (x64) |
| 0.01NHCl- | 0.09 | 0.05 | 0.09 | 0.09 |
| Tw20+Glucose | (x64) | (x128) | (x64) | (x64) |
| 0.01NHCl- | 0.1 | 0.1 | 0.1 | 0.1 |
| Dex70(1hr) | (x64) | (x64) | (x64) | (x64) |
| 0.01NHCl- | 0.1 | 0.1 | 0.1 | 0.1 |
| Dex70+Glu(1hr) | (x64) | (x64) | (x64) | (x64) |

In the reaction system using Tween 20, the antibacterial activities of the obtained polymer particles were at about the same level, regardless of whether glucose was used or not. When compared to the reaction system using dextran, the antibacterial activities of the polymer particles were also at about the same level. The MICs against *Staphylococcus aureus* and *Enterococcus* were 0.05 to 0.1 mg/ml, and it was revealed that the particles exhibited an antibacterial activity equally against sensitive and resistant bacteria.

### Example 4

### Confirmation of Adhesiveness and Bacteriolysis

Gram-positive bacteria were treated with the 0.01NHCl-Dex70+Glucose particles prepared in Production Example 1, and the morphological change of the treated bacteria was observed with an electron microscope. Treatment with the polymer particles was carried out as follows.

The above-described particles were suspended in physiological saline to prepare a particle suspension (6 µg/ml). Using a commercially available 24-well cell culture plate, 1 ml of the particle suspension was added to 10⁵ to 10⁶ cells/1 ml per well of Gram-positive bacterial cells or mammalian cells, and the particles and bacterial cells were suspended together in the well at room temperature for 1 hour. Thereafter, 1 ml/well of physiological saline was added, and the plate was shaken twice or 3 times, followed by removing a washing fluid by aspiration. This washing operation was repeated another twice to carry out the washing 3 times in total. The washed bacterial cells were observed with a scanning election microscope (SEM).

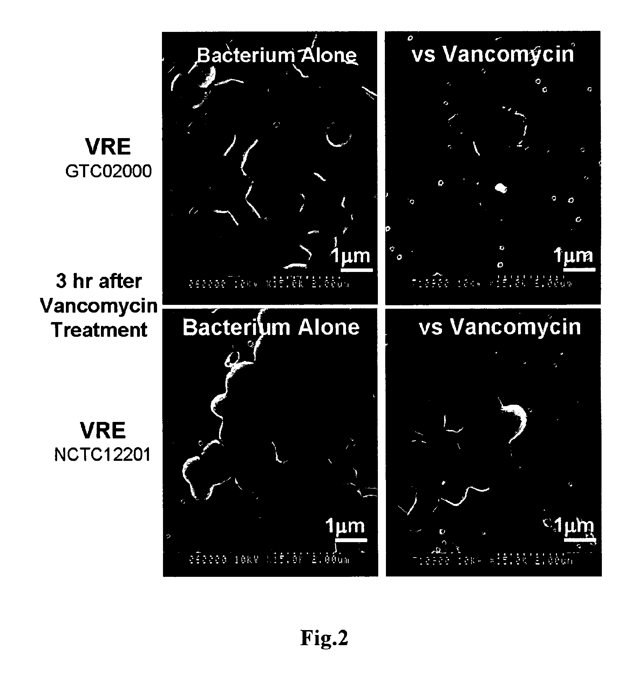

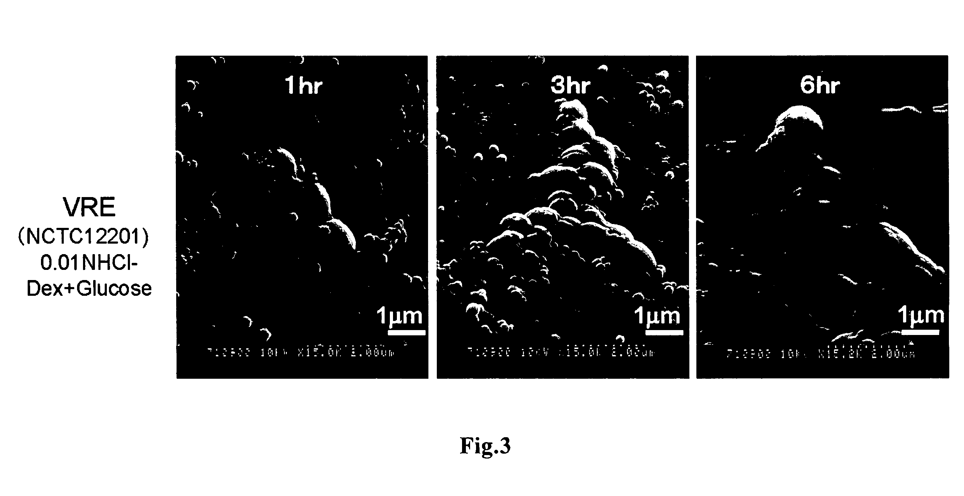

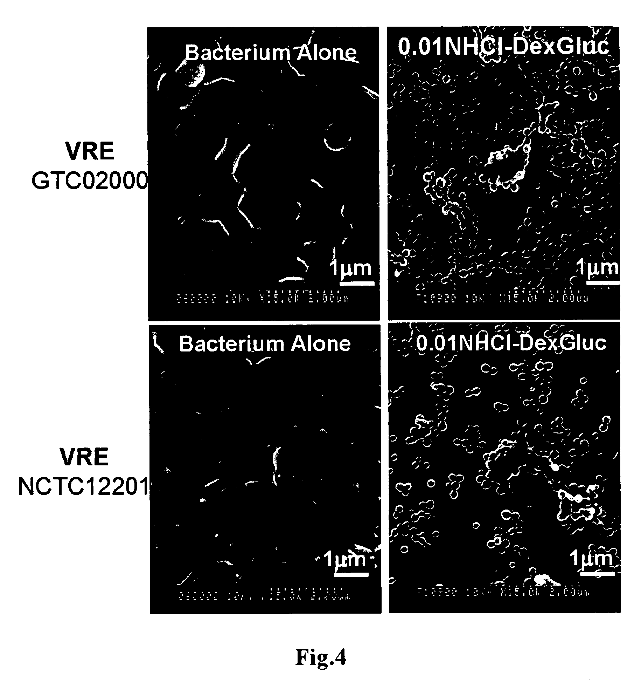

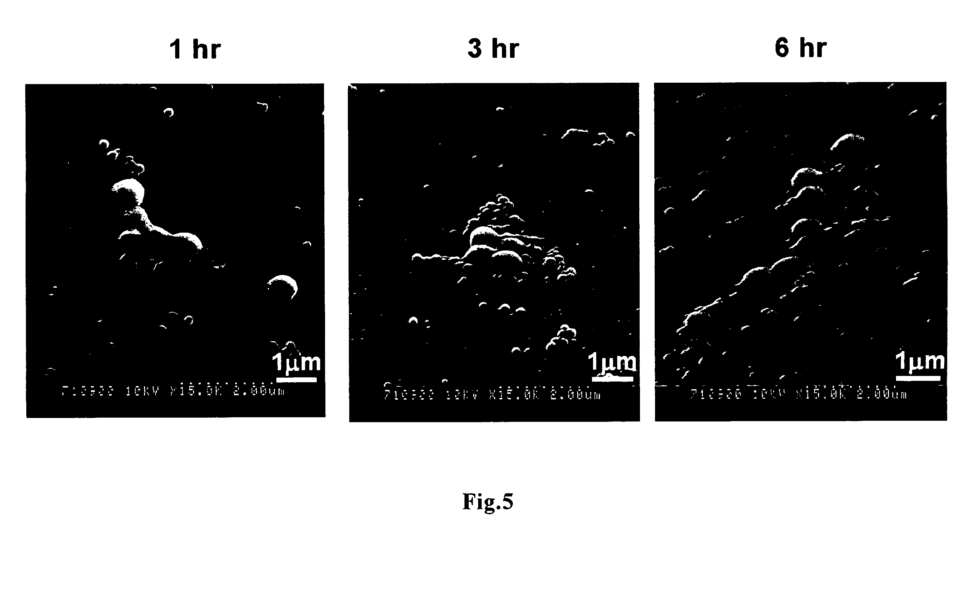

No change was observed when VRE NCTC12201, which is a multidrug-resistant bacterium resistant to vancomycin, was treated with vancomycin (Fig. 2). On the other hand, when VRE NCTC12201 was treated with the above-described polymer particles, it was observed that the particles adhered to the bacterial cell surface and that swelling and lysis of the bacterial cells occurred (Fig. 3). Similarly, in another strain of VRE, adhesion of the particles and bacteriolysis were observed, and no difference was found in the morphological change between the strains (Fig. 4). Moreover, similarly to VRE, adhesion of the polymer particles to the bacterial cell surface and bacteriolysis were also observed in MRSA JCM8703, which is a multidrug-resistant bacterium resistant to methicillin (Fig. 5).

To investigate the adhesiveness to mammalian cells, HeLa cells and CHO cells cultured in a 24-well cell culture plate were treated with the particles and then washed in the same manner as described above, and the cells were observed with SEM. As a result, adhesion of the particles to the cell surface was not observed, indicating that these particles are not adhesive to mammalian cell membrane. In addition, to examine toxicity of the particles to a living body, a subacute toxicity test was carried out in mice according to the Japanese pharmacopoeia. As a result, no signs of toxicity were observed.

### Reference Example: Check of Antibacterial Activity of Used Fluid for Washing Polymer Particles

Using a used washing fluid obtained from the step of washing the polymer particles by centrifugal filtration with a Centriprep filter in Production Examples 1 to 3, the possibility that a component(s) eluted from the polymer particles exhibited the antibacterial activity was investigated. In the same manner as described in Example 1, a used washing fluid obtained from the 4th washing step was examined for an antibacterial activity. As a result, none of the used washing fluids obtained from Production Examples 1 to 3 could inhibit the growth of MSSA and MRSA.

## Claims

1. An antibacterial agent for Gram-positive bacteria, comprising as an effective ingredient particles having a particle diameter of not more than 5 µm, which particles are adhesive to cell wall of Gram-positive bacteria and not adhesive to mammalian cell membrane and substantially do not contain an active antibacterial ingredient effective against Gram-positive bacteria.

2. The antibacterial agent according to claim 1, wherein said particles have a particle diameter of not more than 1 µm.

3. The antibacterial agent according to claim 2, wherein said particles have an average particle diameter of 20 nm to 500 nm.

4. The antibacterial agent according to claim 3, wherein said particles have an average particle diameter of 20 nm to 300 nm.

5. The antibacterial agent according to any one of claims 1 to 4, wherein said particles have a zeta-potential of-3 mV to -80 mV.

6. The antibacterial agent according to any one of claims 1 to 5, wherein said particles are polymer particles whose repeating unit comprises a structure represented by Formula (I) below: 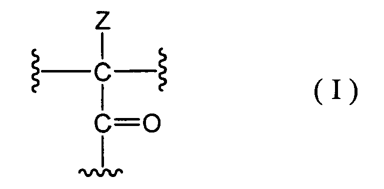 [wherein Z is an electron donating group].

7. The antibacterial agent according to claim 6, wherein in said Formula (I), Z is a cyano group, an amino group or an imino group.

8. The antibacterial agent according to claim 6 or 7, wherein said structure represented by Formula (I) is a structure represented by Formula (II) below: 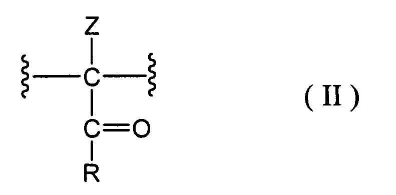 [wherein Z represents the same meaning as described in claim 1; R is hydrogen, a hydroxy group, C₁-C₁₀ alkyl group, C₁-C₁₀ alkoxy group, C₆-C₁₅ aryl group, C₆-C₁₅ aryloxy group, C₇-C₁₆ aralkyl group or a C₇-C₁₆ aralkyloxy group; one or more carbon atoms constituting a carbon chain in R are optionally replaced by nitrogen, sulfur and/or oxygen].

9. The antibacterial agent according to claim 8, wherein in said Formula (II), R is a hydroxy group or a C₁-C₁₀ alkoxy group.

10. The antibacterial agent according to claim 9, wherein in said Formula (II), Z is a cyano group and R is a *n*-butoxy group.

11. The antibacterial agent according to any one of claims 6 to 10, wherein said polymer particles are produced by polymerizing a monomer represented by Formula (III) below: 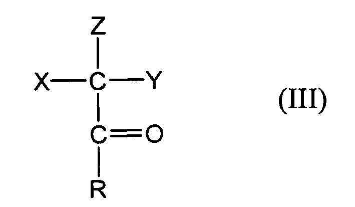 [wherein Z and R represent the same meanings as in said Formula (II); X and Y each independently represent an atomic group having a carbon-carbon double bond in its terminal, or X and Y together form a methylidene group]
in the presence of said monomer and a saccharide(s) having a hydroxy group(s) and/or polysorbate(s).

12. The antibacterial agent according to claim 11, wherein in said Formula (III), Z is a cyano group, an amino group or an imino group; R is a hydroxy group or a C₁-C₁₀ alkoxy group; and X and Y together form a methylidene group.

13. The antibacterial agent according to claim 12, wherein said monomer represented by Formula (III) is n-butyl-2-cyanoacrylate.

14. The antibacterial agent according to any one of claims 11 to 13, wherein said saccharide(s) is(are) at least one selected from the group consisting of glucose, ribose, fructose, mannose, lactose, trehalose, maltose, sucrose, mannan and dextran having an average molecular weight of not less than 70,000.

15. The antibacterial agent according to claim 14, wherein said saccharide(s) is(are) at least one selected from the group consisting of glucose, ribose, lactose, trehalose and dextran having an average molecular weight of not less than 70,000.

16. The antibacterial agent according to any one of claims 11 to 15, wherein said polysorbate is polyoxyethylene sorbitan monolaurate.

17. A method for inhibiting growth of a Gram-positive bacterium/bacteria, comprising bringing a Gram-positive bacterium/bacteria to be inhibited into contact with an effective amount of particles having a particle diameter of not more than 5 µm, which particles are adhesive to cell wall of Gram-positive bacteria and not adhesive to mammalian cell membrane and substantially do not contain an active antibacterial ingredient effective against Gram-positive bacteria.

18. Use of particles having a particle diameter of not more than 5 µm, which particles are adhesive to cell wall of Gram-positive bacteria and not adhesive to mammalian cell membrane and substantially do not contain an active antibacterial ingredient effective against Gram-positive bacteria, for the production of an antibacterial agent for Gram-positive bacteria.
